# EUROPÄISCHE PATENTSCHRIFT

(11) **EP 0 807 086 B1**
(45) Veröffentlichungstag und Bekanntmachung des Hinweises auf die Patenterteilung: **22.09.1999**
(21) Anmeldenummer: 96901690.6
(22) Anmeldetag: 30.01.1996
(51) Int. Cl.: C01F 7/00, C01B 13/32, B01J 32/00, C04B 35/443, B01J 41/10, A61K 33/08, A61K 33/24

(54) **VERFAHREN ZUR HERSTELLUNG VON HYDROTALCITEN**
PROCESS FOR PRODUCING HYDROTALCITES
PROCEDE DE PREPARATION D'HYDROTALCITES

(30) Priorität: 03.02.1995 DE 19503522
(43) Veröffentlichungstag der Anmeldung: 19.11.1997
(73) Patentinhaber: RWE-DEA AKTIENGESELLSCHAFT FÜR MINERALOEL UND CHEMIE, 22297 Hamburg (DE)
(72) Erfinder: DIBLITZ, Klaus, D-22869 Schenefeld (DE); NOWECK, Klaus, D-25541 Brunsbüttel (DE); SCHIEFLER, Jan, D-22605 Hamburg (DE); BRASCH, Andrea, D-25704 Meldorf (DE)
(74) Vertreter: Schupfner, Gerhard D.
(86) Internationale Anmeldenummer: DE9600149
(87) Internationale Veröffentlichungsnummer: WO9623727

(56) Entgegenhaltungen:
- EP-A- 0 103 034
- EP-A- 0 207 811
- WO-A-93/21961
- WO-A-93/22237
- US-A- 3 650 704
- US-A- 5 084 209
- CATALYSIS TODAY, Bd. 11, 1.Januar 1991, Seiten 173-291, XP000537043 CAVANI F ET AL: "HYDROTALCITE-TYPE ANIONIC CLAYS: PREPARATION, PROPERTIES AND APPLICATIONS"

## Beschreibung

Die Erfindung betrifft die Herstellung von Hydrotalciten. Diese sind Metallhydroxide mit Schichtstruktur und gehören zur Gruppe der anionischen Tonminerale. Weiter beinhaltet die Erfindung die durch Kalzinieren der erfindungsgemäß hergestellten Metallhydroxide hergestellten Metalloxide.

Metallhydroxide sind wichtige Vorstufen zur Herstellung von Metalloxiden, wie sie etwa als Rohstoffe für Feuerfestmaterialien, keramische Werkstoffe und Trägermaterialien für heterogene Katalysatoren eingesetzt werden. In der Natur kommen Metallhydroxide überwiegend in Form von gemischten Metallhydroxiden vor. So gibt es eine Vielzahl von Tonmineralien, die durch ihren schichtförmigen Aufbau charakterisiert werden können. Am häufigsten treten kationische Tonmineralien auf, bei denen sich Kationen (z. B. Na⁺, Ca²⁺ etc.) zwischen den negativ geladenen Schichten der Metallhydroxide befinden. Weit weniger häufig sind dagegen anionische Tonmineralien, bei den sich zwischen den positiv geladenen Metallhydroxidschichten Anionen befinden. Viele dieser anionischen Tonmineralien bestehen aus den Hydroxiden der Hauptgruppenmetalle Magnesium und Aluminium und Hydroxiden der Übergangsmetalle wie Nickel, Chrom, Zink etc. Die Struktur solcher Tonminerale läßt sich von der Brucit-Struktur vom Magnesiumhydroxid Mg(OH)₂ ableiten. Hierbei sind einige der divalenten Mg(OH)₆⁴⁻ Oktaeder durch Al(OH)₆³⁻ - Oktaeder ersetzt. Als Beispiele seien Meixnerit mit der idealisierten Formel der Einheitszelle Mg₆Al₂(OH)₁₈ * 4 H₂O und Hydrotalcit Mg₆Al₂(OH)₁₆CO₃ * 4 H₂O genannt. Das Verhältnis von Magnesium zu Aluminium kann nach dem Stand der Technik zwischen 1.7 und 4 variieren. Die Metallhydroxidoktaeder sind über die Kanten zu Schichten verbunden. Zwischen den Schichten befindet sich Wasser sowie die zum Erreichen des Ladungsausgleichs erforderlichen interstitiellen Anionen. Die Anionen können einfacher Natur wie z.B. OH⁻, CO₃²⁻, Cl⁻ oder SO₄²⁻ sein, sie können aber auch komplexerer Natur sein wie etwa voluminöse organische oder anorganische Anionen. Diese wurden bisher durch den Austausch einfacher Anionen oder Säurebehandlung in Gegenwart der gewünschten Anionen in die Schichten eingebaut.

Verfahren zur Herstellung von anionischen, schichtförmig aufgebauten Tonmineralen sind vielfältig in der Literatur beschrieben. Dabei werden als Ausgangssubstanzen stets Salze der Metalle in Lösung gebracht und bei bestimmten pH-Bereichen miteinander vermengt. Beispiele sind US-PS 4,539,306 zur Herstellung von Hydrotalciten für pharmazeutische Anwendungen sowie W. T. Reichle in Journal of Catalysis 1985, 94, 547-557 und J. G. Nunan et al. in Inorganic Chemistry 1989, 28, 3868-3874. Misra et al. beschreibt in der US-PS 5,075,089 die Herstellung von Hydrotalciten mit schichtaufweitenden interstitiellen Anionen durch Austausch der Anionen bei erhöhten Temperaturen. Beispiele für den Einbau voluminöser organischer Anionen durch Anionenaustausch findet man in G. Lagaly et al., Inorganic Chemistry 1990, 29, 5201-5207. Miyata et al. beschreiben in Clay and Minerals, 1977, 25, 14-18 die Herstellung von Magnesium/Aluminium-Hydrotalciten durch Mischen von Lösungen der Salze MgCl₂ und Al₂(SO₄)₃ und einer NaOH-Lösung. In der EP-A1- 0 536 879 wird die Herstellung von anionischen, schichtförmig aufgebauten Tonmineralen mit schichtaufweitenden, anorganischen Anionen wie B(OH)⁴⁻, V₄O₁₂⁴⁻, V₂O₇⁴⁻ oder HV₂O₇³⁻ beschrieben. Auch hier werden Lösungen der Metallsalze unter definierten pH-Bedingungen mit Lösungen der einzubauenden Salze versetzt. Zur Anwendung von anionischen, schichtförmig aufgebauten Tonmineralen als Katalysatoren findet man Beispiele in der US-PS 4,774,212 und der US-PS 4,843,168 sowie der EP-A1- 0 536 879 und M. Drezdon, ACS-Symp. Ser., (Novel Mater. Heterog. Catal.), 1990, 437, 140-148.

WO-A-93 21 961 beschreibt ein Verfahren zur Herstellung von schichtförmigen, gemischten Metallhydroxiden durch kontrollierte Hydrolyse von Metalloxiden in einem wasserfreien organischen Lösungsmittel mit stöchiometrischen Mengen Wasser. Die hierbei erhaltenen Metallhydroxide sind gelförmige Zusammensetzungen zum Einsatz als Farbträger zur Anwendung in Farblaser-Systemen. Diese Metallhydroxide weisen gemäß Seite 4 unten der Entgegenhaltung die folgende Zusammensetzung auf:

MₘD_{d}T(OH)_{(5-m+d)}*nH2O

mit M, D und T als ein-, zwei- und dreiwertige Metalle, m=0-1, d=0-1, wobei m + d ≠ O sein sollen. Bei diesen so hergestellten Metallhydroxiden handelt es sich nicht um chemische Verbindungen, sondern gelförmige Zusammensetzungen, die als Vorstufe zur Herstellung von Metalloxiden definierter Struktur gemäß der Aufgabenstellung der vorliegenden Erfindung ungeeignet sind.

Einer weiten Verbreitung von anionischen, schichtförmig aufgebauten Tonmineralen stand bisher entgegen, daß bedingt durch deren Herstellung aus Lösungen von Metallsalzen nur eine zeitaufwendige diskontinuierliche Syntheseroute bestand.

Weiter ist allgemein bekannt, daß die Reinheit von Katalysatoren ein wichtiges Kriterium darstellt. Insbesondere sind Verunreinigungen durch Alkali- und Erdalkalimetalle störend. Solche Verunreinigungen sind jedoch beim Einsatz von Metallsalzen nicht oder nur durch extrem hohen Aufwand und dadurch hohen Kosten zu vermeiden. Weiter ist bisher kein Verfahren bekannt, hydrotalcitartige Tonminerale, welche nur OH⁻ Ionen in den Schichten aufweisen, ohne einen zusätzlichen nachfolgenden Ionenaustausch herzustellen.

Die für die katalytische Charakteristik ausschlaggebende Eigenschaft solcher Tonminerale ist deren Basizität. Diese wird nach dem Stand der Technik wesentlich vom Verhältnis Magnesium zu Aluminium bestimmt [siehe dazu A. L. McKenzie, C. T. Fishel und R. J. Davis, J. Catal., 138 (1992) 547]. Zur Anpassung der katalytischen Charakteristik eines Katalysators ist daher eine möglichst breite Variation des Verhältnisses von Magnesium zu Aluminium wünschenswert. Es ist ebenfalls bisher nicht bekannt, schichtförmige, anionische Tonminerale mit einem Verhältnis von Magnesium zu Aluminium kleiner als 1,7 herzustellen.

Der vorliegenden Erfindung liegt die Aufgabe zugrunde ein Verfahren zur Herstellung von anionischen, schichtförmig aufgebauten Tonmineralen zu entwickeln, welches die folgenden Vorteile bietet:
- Die Synthese soll zeitsparend und kontinuierlich sowie diskontinuierlich durchführbar sein.
- Es sollen preisgünstige und ohne weiteres verfügbare Ausgangsmaterialien verwendet werden.
- Die so hergestellten anionischen, schichtförmig aufgebauten Tonminerale sollen eine hohe Reinheit und einen geringen Alkaligehalt besitzen.
- Es sollen auch solche anionischen, schichtförmig aufgebauten Tonminerale darstellbar sein, welche als interstitielle Anionen nur Hydroxidionen aufweisen.
- Die anionischen, schichtförmig aufgebauten Tonminerale sollen die für die katalytische Verwendbarkeit notwendigen großen Porenvolumina und Oberflächen besitzen.
- Es sollen anionische, schichtförmige Tonminerale mit einem Verhältnis von Magnesium zu Aluminium von kleiner 1,7 herstellbar sein.

Diese Aufgabe wird überraschenderweise erfindungsgemäß gelöst durch ein Verfahren zur Herstellung von Hydrotalciten von hoher Reinheit, die anionische, schichtförmig aufgebaute gemischte Metallhydroxide gemäß der allgemeinen Formel

M²⁺₂ₓM³⁺₂(OH)₄ₓ₊₄Aⁿ⁻_{2/n}•zH₂O

sind, worin x = von 0,5 bis 10 in 0,5 Schritten reicht, A für ein interstitielles Anion steht, n die Ladung des interstitiellen Anions ist, die bis zu 8, üblicherweise bis zu 4 beträgt, und z in ganzen Zahlen von 1 bis 6, insbesondere von 2 bis 4 reicht, wobei man
(A) Metallalkoholat-Mischungen einsetzt, die aus zumindest einem oder mehreren zweiwertigen Metallen und zumindest einem oder mehreren dreiwertigen Metallen und aus mono-, di- oder trivalenten C1-bis C40- Alkoholaten bestehen, wobei die zweiwertigen und dreiwertigen Metallalkoholate im wesentlichen in einem molaren Verhältnis eingesetzt werden, daß der Stöchiometrie einer Zielverbindung gemäß obiger Summenformel entspricht und
(B) man das erhaltene Alkoholatgemisch mit Wasser hydrolisiert, wobei das Hydrolysewasser überstöchiometrisch in Bezug auf die reaktiven Valenzen der eingesetzten Metalle eingesetzt wird.
Durch Kalcinierung kann daraus das entsprechende gemischte Metalloxid hergestellt werden.

Die Metallalkoholate sind durch Umsetzung von Metallen, die die Oxidationstufe +II oder +III ausbilden können und Alkoholate zu bilden vermögen, mit mono-, di- oder trivalenten C1-bis C40- Alkoholen erhältlich, wobei die Herstellung der Metallalkoholate
(A) durch gemeinsames Vorlegen der Metalle und Zugabe des Alkohols oder
(B) getrennte Herstellung der Metallalkoholate ggf. mit unterschiedlichen Alkoholat-Resten oder
(C) nacheinander durch Vorlegen des einen Metalls, Zugabe des Alkohols, Zugabe des nächsten Metalls und ggf. erneuter Alkoholzugabe
erfolgt.

Zur Herstellung der Alkoholate können als zweiwertige Metalle Mg, Zn, Cu, Ni, Co, Mn, Ca und/oder Fe und als dreiwertige Metalle Al, Fe, Co, Mn, La, Ce und/oder Cr eingesetzt werden.

Als dritte oder weitere Metallkomponente können vor oder während der Hydrolyse beliebige wasserlösliche zweiwertige oder dreiwertige Metalle als Metallsalze zugesetzt werden, wobei die Metallsalze gegenüber den Metallalkoholaten im molaren Unterschuß verwendet werden.

Die Metallalkoholate werden so hergestellt, daß sie in einem molaren Verhältnis von divalenten zu trivalenten Metallalkoholaten von 1:2 bis 10:1 vorliegen und anschließend hydrolisiert. Zur Abtrennung unlöslicher Komponenten kann das Alkoholat / Alkoholatgemisch vor der Hydrolyse filtriert werden.

Als Alkohole kommen mono-, di- und trivalente mit Kettenlängen von C₁ bis C₄₀ in Betracht, sie können verzweigt, unverzweigt oder ringförmig sein. Bevorzugt sind verzweigte und unverzweigte Alkohole mit Kettenlängen zwischen C₄ und C₂₀, besonders bevorzugt mit Kettenlängen von C₆ bis C₁₄. Zur erfindungsgemäßen Herstellung von anionischen, schichtförmigen Tonmineralen können die Metallalkoxide aus gleichen oder Mischungen von Alkoholen hergestellt werden.

Zur Herstellung hochreiner Tonminerale wird das zur Hydrolyse verwendete Wasser über Ionenaustauscher oder durch mehrfache Destillation gereinigt. Dem Hydrolysewasser können Hydroxid-Anionen und/oder beliebige wasserlösliche Anionen hinzugefügt sein. Als organische Anionen seien genannt insbesondere Alkoholatanionen aber auch Alkylethersulfate , Arylethersulfate und/oder Glykolethersulfate; und/oder als anorganische Anionen insbesondere Carbonat, Hydrogencarbonat, Nitrat, Chlorid, Sulfat, B(OH)₄⁻ und/oder Polyoxometallanionen wie Mo₇O₂₄⁶⁻ oder V₁₀O₂₈⁶⁻. Als Gegenion wird bevorzugt NH₄⁺ verwendet. Die Anionen werden als interstitielle Anionen in dem bei der Hydrolyse enstehenden schichtförmigen Tonmineralien in das Gitter eingebaut. Es ist jedoch auch möglich, Anionen nachträglich durch Anionenaustausch als interstitielle Anionen in die anionischen, schichtförmigen Tonminerale einzubauen.

Der pH-Bereich des Hydrolysewasser kann von 0 bis 14 reichen, vorzugsweise von 1 bis 13. Die Temperatur des Hydrolysewassers kann von 5 bis 98 °C reichen, vorzugsweise von 20 bis 95 °C und besonders bevorzugt von 30 bis 90 °C.

Die erfindungsgemäß hergestellten Hydrotalcite weisen Schichtabstände (d-Werte) von größer > 7 Å auf, gemessen am d(003)-Reflex. Tabelle I gibt die Zusammensetzung und die physikalischen Daten von Beispielen der erfindungsgemäß hergestellten Mg(Zn)/Al-Tonminerale wieder.

**Tabelle 1:**

| **Verbindung** | **Al/M**^{**2+**} | **Formel** | **Oberfläche** ^{**a**} | **Porenvolumen** ^{**a**} | **Porenadius** ^{**a**} | **Kristallitgröße** ^{**b**} | **d-Wert** ^{**b**} |
|---|---|---|---|---|---|---|---|
| | | | [m²/g | [ml/g] | [Å] | [Å] | [Å] |
| 1 | 2:1 | MgAl₂(OH)₈*4 H₂O | 244 | 0,26 | 23 | | 7,72 |
| 2 | 1:1 | Mg₂Al₂(OH)₁₀*4 H₂O | 217 | 0,43 | 26 | 210 | 7,78 |
| 3 | 1:1,25 | Mg₅Al₄(OH)₂₂*4 H₂O | 230 | 0,13 | 29 | | 8,22 |
| 4 | 1:1,5 | Mg₃Al₂(OH)₁₂*4 H₂O | 276 | 0,78 | 51 | | 7,82 |
| 5 | 1:2 | Mg₄Al₂(OH)₁₄*4 H₂O | 283 | 0,69 | 43 | 230 | 7,79 |
| 6 | 1:3 | Mg₆Al₂(OH)₁₈*4 H₂O | 190 | 0,39 | 161 | | 7,73 |
| 7 | 1:5 | Mg₁₀Al₂(OH)₂₆*4 H₂O | 197 | 0,29 | 25 | | |
| 8 | 1:10 | Mg₂₀Al₂(OH)₄₆*4 H₂O | 181 | 0,35 | 32 | 420 | |
| 9 | 1:3 | Mg₆Al₂(OH)₁₆(NO₃)₂*4 H₂O | 180 | 0,32 | 29 | | 8,73 |
| 10 | 1:3 | Mg₆Al₂(OH)₁₆(HCO₃)₂*4 H₂O | 190 | 0,76 | 174 | 400 | 7,79 |
| 11 | 1:3 | Mg₆Al₂(OH)₁₆CO₃*4 H₂O | 182 | 0,77 | 217 | | 7,77 |
| 12 | 1:3 | Mg₁₈Al₆(OH)₄₈(Mo₇O₂₄)*4 H₂O | 26 | 0,06 | 22 | | 9,26 |
| 13 | 1:3 | Mg₆Al₂(OH)₁₆(C₆H₆O₇)*4 H₂O | 330 | 0,29 | 19 | | 11,23 |
| 14 | 1:3 | Mg₆Al₂(OH)₁₆(C₃H₅O₂)₂*4 H₂O | 236 | 0,57 | 37 | 200 | 13.22 |
| 15 | 1:1,5 | Mg₃Al₂(OH)₁₀(NO₃)₂*4 H₂O | 145 | 0,24 | 26 | | 8,98 |
| 16 | 1:1,5 | Mg₃Al₂(OH)₁₀(HCO₃)₂*4 H₂O | 255 | 1,03 | 71 | | 7.59 |
| 17 | 1:1,5 | Mg₃Al₂(OH)₁₀CO₃*4 H₂O | 268 | 1,15 | 101 | | 7,59 |
| 18 | 1:1,5 | Mg₃Al₂(OH)₈(C₆H₆O₇)₂*4 H₂O | 375 | 0,06 | 37 | | amorph |
| 19 | 1:3 | Zn₆Al₂(OH)₁₈*4 H₂O | 180 | 0,44 | 100 | | amorph |

| | | | | | | | |
|---|---|---|---|---|---|---|---|
| *a = aktiviert bei 550 °C für 3 h ,* | | | | | | | |
| *b = gemessen am Hydrat* | | | | | | | |

Metallhydroxide sind wichtige Vorstufen zur Herstellung von Metalloxiden. Die erfindungsgemäß hergestellten Metalloxide finden als hochreine Rohstoffe Verwendung für Feuerfestmaterialien, keramische Werkstoffe und Trägermaterialien für Katalysatoren. Die Metallhydroxide können als hochreine anorganische Ionentauscher und Molekularsiebe Verwendung finden, desweiteren als Zusatzstoffe zu Zahncreme als Antikariesschutz oder als Antiazidotika in der Pharmazie sowie als Additive für Kunststoffe, zB. als Flammschutzmittel und Vergilbungsstabilisatoren in PVC.

Die schichtförmigen, anionischen Tonminerale werden in hoher Reinheit hergestellt. Dies wird durch die erfindungsgemäße Umsetzung der Metalle mit Alkoholen zu Alkoholaten und nachfolgende Reinigung der Alkoholate etwa durch Filtration erreicht. Tabelle 2 enthält die Analysendaten der erfindungsgemäß hergestellten Verbindungen im Vergleich zu den Ausgangsmetallen und von durch Umsetzung von Metallsalzen hergestellten Vergleichsprodukten. Das Vergleichsprodukt A (VP A) wurde aus Metallsalzen, Nitratsalze in p.a.-Qualität, wie in der Literatur beschrieben erhalten. Vergleichsprodukt B (VP B) wurde durch Umsetzung von Metallhydroxiden hergestellt.

**Tabelle 2:**

| *Analysendaten der Spurenelementbestimmung mittels ICP* | | | | | | | | | | |
|---|---|---|---|---|---|---|---|---|---|---|
| **Substanz** | **Si** | **Fe** | **Mn** | **Ti** | **Zn** | **Ga** | **Na** | **Ca** | **Cu** | **Pb** |
| | [ppm] | [ppm] | [ppm] | [ppm] | [ppm] | [ppm] | [ppm] | [ppm] | [ppm] | [ppm] |
| Mg-Pulver | 64 | 204 | 129 | < 1 | 13 | < 5 | 86 | 11 | 10 | 75 |
| Mg-Granulat | 46 | 286 | 8 | 3 | 23 | < 5 | 23 | 9 | 3 | 90 |
| Al-Nadeln | 980 | 2387 | 45 | 36 | 147 | 98 | 25 | 8 | 22 | 22 |
| VPA | 303 | 84 | 3 | 2 | 4 | 6 | 890 | 114 | < 2 | 46 |
| VP B | 620 | 300 | 11 | < 1 | 11 | < 5 | 40 | 1650 | < 2 | 40 |
| 6 | 50 | 68 | 8 | < 1 | 15 | < 5 | 5 | 8 | < 2 | < 10 |
| 3 | 62 | 70 | 15 | < 1 | 5 | < 5 | 6 | 17 | < 2 | 40 |
| 1 | 60 | 75 | 19 | < 2 | 25 | < 5 | 6 | 11 | < 2 | 20 |
| 9 | 58 | 72 | 8 | 1 | 15 | < 5 | 6 | 20 | 4 | 10 |

Zur Herstellung der erfindungsgemäßen Verbindungen wurden die in Tabelle 2 aufgeführten Magnesium-Pulver bzw. Granulate und Aluminium-Nadeln eingesetzt. Die in Tabelle 2 gelisteten Daten zeigen, daß die erfindungsgemäß hergestellten Verbindungen die für viele Einsatzzwecke erforderliche hohe Reinheit besitzen. Besonders vorteilhaft ist die deutliche Reduzierung der Gehalte an Alkali- und Erdalkalimetallen (Natrium und Calcium) sowie des Silizium- und Eisengehalts, da diese Elemente gerade bei Anwendungen in der Katalyse sehr nachteilig sind.

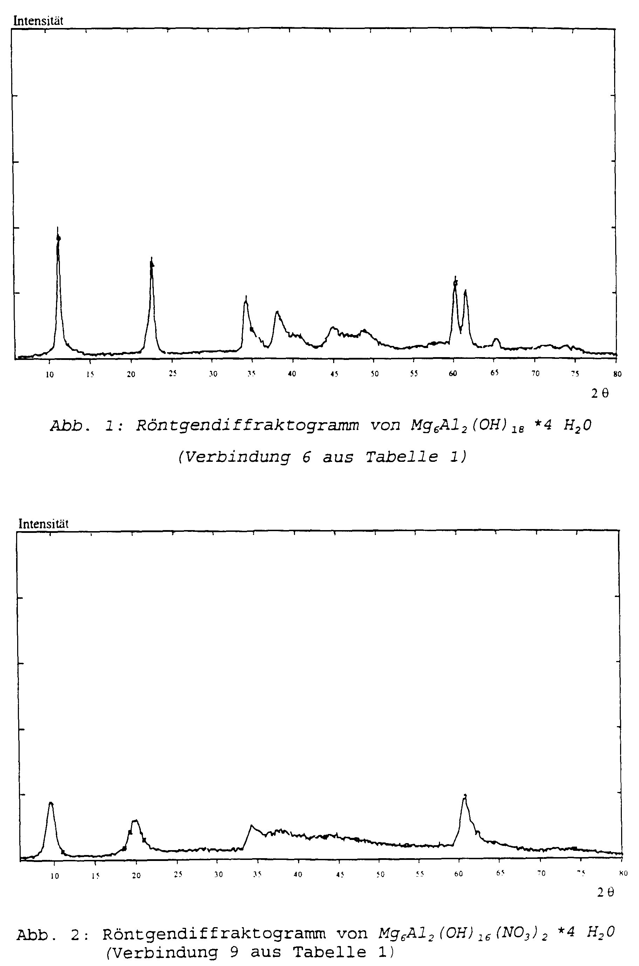

Die erfindungsgemäßen hergestellten Verbindungen weisen die typischen Röntgendiffraktogramme in Abb. 1 und Abb. 2 auf. Zum Vergleich dient das Röntgendiffraktogramm Abb. 3 einer Verbindung, welche aus einer Lösung von Aluminiumhydroxid und Magnesiumhydroxid hergestellt wurde. Hier liegt unverändert Aluminiumhydroxid neben Magnesiumhydroxid vor, es ist keine Bildung von Tonmineralen zu beobachten.

Aus den erfindungsgemäß hergestellten Verbindungen lassen sich durch Kalzinieren gemischte Metalloxide herstellen. Zum Kalzinieren wurden die erfindungsgemäßen Verbindungen in einen Ofen bei Temperaturen zwischen 550 °C und 1500 °C über einen Zeitraum von 3 h bis 24 h verbracht. Die so hergestellten gemischten Metalloxide weisen die gleiche hohe Reinheit wie die erfindungsgemäßen gemischten Metallhydroxide auf.

In Tabelle 3 sind die Oberflächen von kalzinierten Verbindungen bei verschiedenen Kalzinierungstemperaturen aufgeführt. Um die hohe Oberflächenstabilität der erfindungsgemäß hergestellten Verbindungen im Vergleich zu einem durch Mischen der Metallhydroxide hergestellten Produkt zu verdeutlichen, wurde ein Vergleichsprodukt B (VP B) unter gleichen Bedingungen kalziniert. Das Vergleichsprodukt B besitzt das gleiche Verhältnis der Metalle wie das erfindungsgemäß hergestellte Mg₆Al₂(OH)₁₈*4 H₂O.

**Tabelle 3:**

| *Oberflächen der kalzinierten Verbindungen* | | | | | |
|---|---|---|---|---|---|
| **Verbindung / Oberfläche [m**^{**2**}**/g]** | **3h / 550°C** | **3h / 750°C** | **3h / 950°C** | **3h / 1200°C** | **3h / 1500°C** |
| Mg₆Al₂(OH)₁₈*4 H₂O | 190 | 132 | 106 | 33 | 6 |
| VP B | 138 | 73 | 43 | 32 | 1 |

In Tabelle 4 werden erfindungsgemäß hergestellte Metallhydroxide sowie die jeweils durch Kalzinieren daraus hergestellten Metalloxide aufgeführt.

**Tabelle 4:**

| *Metalloxide aus Metallhydroxidvorstufen* | | |
|---|---|---|
| Verbindung | Hydroxidvorstufe | kalziniertes Produkt |
| 1 | MgAl₂(OH)₈*4 H₂O | MgAl₂O₄ |
| 2 | Mg₂Al₂(OH)₁₀*4 H₂O | Mg₂Al₂O₅ |
| 3 | Mg₅Al₄(OH)₂₂*4 H₂O | Mg₅Al₄O₁₁ |
| 6 | Mg₆Al₂(OH)₁₈*4 H₂O | Mg₆Al₂O₉ |

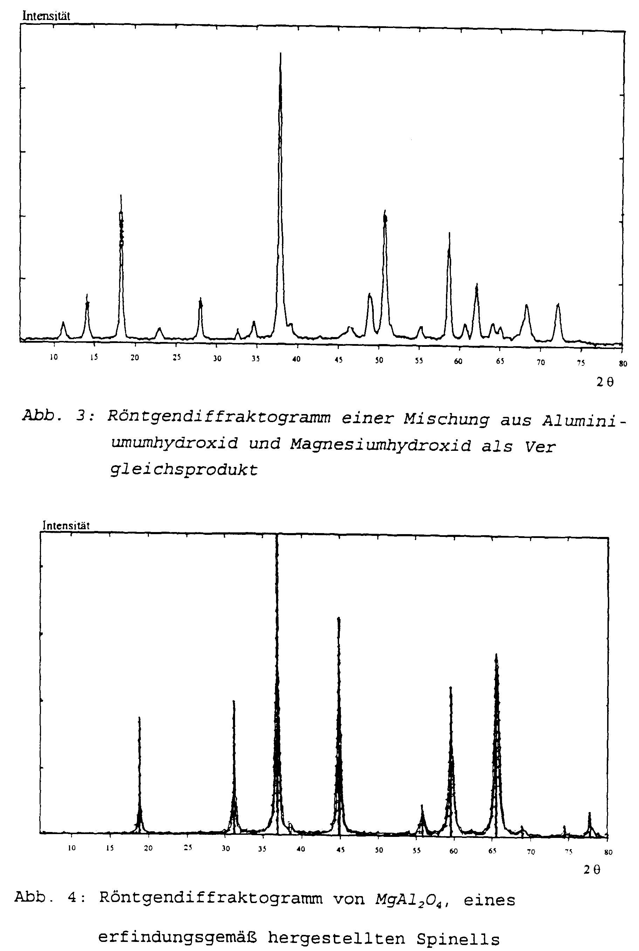

Abb. 4 zeigt das Röntgendiffraktogramm eines aus Verbindung 1 (siehe Tabelle 1) hergestellten Spinells. Das Strichspektrum in Abb. 4 gibt zum Vergleich das Röntgendiffraktogramm aus der JCPDS-Kartei (Eintrag Nr. 21-1152 MgAl₂O₄, Spinell, syn) wieder. Der Vergleich zeigt, daß durch Kalzinieren der erfindungsgemäßen Verbindung 1 ein phasenreiner Spinell MgAl₂O₄ entsteht.

### Beispiele

### Allgemein

Zur Analyse der erfindungsgemäß hergestellten Verbindungen werden die Metallverhältnisse mittels induktiv gekoppelter Plasmaspektroskopie bestimmt. Ebenfalls nach dieser Methode werden die Verunreinigungen bestimmt. Die Bestimmung der kristallinen Phasen sowie der Kristallitgrößen und des Schichtebenenabstands am d(001) bzw. d(003)-Reflexes erfolgte durch Pulverdiffraktometrie. Der Nachweis der interstitiellen Ionen (OH⁻, HCO₃⁻, NO₃⁻ etc.) erfolgte durch die Thermogravimetrie, nach dieser Methode wurde ebenfalls der Gehalt an Kristallwasser bestimmt. Alle diesbezüglichen Mengenangaben sind in Gewichtsprozent. Oberflächen und Porenradien wurden mittels BET (3-Punkt Methode) ermittelt, Porenvolumina zusätzlich mittels Quecksilberporosimetrie. Zur Bestimmung des Wassergehaltes und der Menge der in den Schichten eingelagerten Ionen wird die Thermogravimetrie eingesetzt. Zum Kalzinieren wurden die erfindungsgemäßen Verbindungen in einem Muffelofen Temperaturen zwischen 550 °C und 1500 °C ausgesetzt.

### Beispiel 1 Verbindung 1 aus Tabelle 1

In einen 1000 ml Dreihalskolben werden 15,5 g Aluminium-Nadeln und 6,5 g Magnesium-Granulate vorgelegt. Dazu gibt man 239,6 g Hexanol. Die Mischung wird erhitzt. Bei ca. 160 °C beginnt die Umsetzung der Metalle mit dem Hexanol, erkennbar an der Entwicklung von Wasserstoff und einem Temperaturanstieg auf ca. 230 °C. Man tropft nun mittels eines Tropftrichters weitere 534,5 g Hexanol über einen Zeitraum von 60 min. zu. Das Reaktionsgemisch wird bei 90 °C filtriert. Das Filtrat wird in drei aliquoten Teilen in einer Vorlage bestehend aus 723,5 g deionisiertem Wasser (enthält 0,2 Gew.% Ammoniak) hydrolysiert. Es entsteht sofort ein weißer Niederschlag. Der überstehende Alkohol wird abdekantiert, geringe Mengen des in der Wasserphase gelösten Alkohols können durch eine Wasserdampfdestillation abgestrippt werden. Der so erhaltene Slurry wird sprühgetrocknet. Die Ausbeute beträgt entsprechend 98 % der Theorie. Die ICP-Analyse ergibt ein Verhältnis Aluminiumoxid:Magnesiumoxid wie 73,2 %:26,8 % (berechnet 73%: 27 %). Die Auswertung der Thermogravimetrie ergibt: Rückstand 50,3 % entsprechend MgAl₂O₄ (theor. 49,7 %), - 4 H₂O (Kristallwasser) 24,9 % (theor. 25,2 %), - 4 H₂O 25,4 % (theor. 25,2 %).

### Beispiel 2 Verbindung 6 aus Tabelle 1

In einen 1000 ml Dreihalskolben werden 7,9 g Aluminium-Nadeln und 21,1 g Magnesium-Granulate vorgelegt. Dazu gibt man 122 g Hexanol. Die Mischung wird erhitzt. Bei ca. 160°C beginnt die Umsetzung der Metalle mit dem Hexanol, erkennbar an der Entwicklung von Wasserstoff und einem Temperaturanstieg auf ca. 230 °C. Man tropft nun mittels eines Tropftrichters weitere 574 g Hexanol über einen Zeitraum von 60 min. zu. Das Reaktionsgemisch wird bei 90°C filtriert. Das Filtrat wird in drei aliquoten Teilen in einer Vorlage bestehend aus 943 g deionisiertem Wasser (enthält 0,2 Gew.% Ammoniak) hydrolysiert. Es entsteht sofort ein weißer Niederschlag. Der überstehende Alkohol wird abdekantiert, geringe Mengen des in der Wasserphase gelösten Alkohols können durch eine Wasserdampfdestillation abgestrippt werden. Der so erhaltene Slurry wird sprühgetrocknet. Die Ausbeute beträgt entsprechend 98 % der Theorie. Die ICP-Analyse ergibt ein Verhältnis Aluminiumoxid:Magnesiumoxid wie 32,2 %:67,8 % (berechnet 30% : 70%). Die Auswertung der Thermogravimetrie ergibt: Rückstand 60,2 % entsprechend Mg₆Al₂O₉ (theor. 59,5 %), - 4 H₂O (Kristallwasser) 13,2 % (theor. 12,5 %), - 9 H₂O 27,0 % (theor. 28,0 %).

### Beispiel 3 Verbindung 9 aus Tabelle 1

In einen 1000 ml Dreihalskolben werden 7,9 g Aluminium-Nadeln und 21,1 g Magnesium-Granulate vorgelegt. Dazu gibt man 117 g Hexanol. Die Mischung wird erhitzt. Bei ca. 160°C beginnt die Umsetzung der Metalle mit dem Hexanol, erkennbar an der Entwicklung von Wasserstoff und einem Temperaturanstieg auf ca. 230 °C. Man tropft nun mittels eines Tropftrichters weitere 574 g Hexanol über einen Zeitraum von 60 min. zu. Das Reaktionsgemisch wird bei 90°C filtriert. Das Filtrat wird in drei aliquoten Teilen in einer Vorlage bestehend aus 876 g deionisiertem Wasser und darin gelösten 32,2 g Ammoniumnitrat hydrolysiert. Es entsteht sofort ein weißer Niederschlag. Der überstehende Alkohol wird abdekantiert, geringe Mengen des in der Wasserphase gelösten Alkohols können durch eine Wasserdampfdestillation abgestrippt werden. Der so erhaltene Slurry wird sprühgetrocknet. Die Ausbeute beträgt entsprechend 98% der Theorie. Die ICP-Analyse ergibt ein Verhältnis Aluminiumoxid:Magnesiumoxid wie 30,8 %:69,2 % (berechnet 30 %:70 %). Die Auswertung der Thermogravimetrie ergibt: Rückstand 52,5 % entsprechend Mg₆Al₂O₉ (theor. 51,5 %), - 4 H₂O (Kristallwasser) 10,6 % (theor. 10,8 %), - 5 H₂O 12,5 % (theor. 13,5 %), - 2 H₂O - 2 HNO₃ 24,9 % (theor. 24,3 %).

### Beispiel 4 Verbindung 11 aus Tabelle 1

In einen 1000 ml Dreihalskolben werden 7,9 g Aluminium-Nadeln und 21,1 g Magnesium-Granulate vorgelegt. Dazu gibt man 118 g Hexanol. Die Mischung wird erhitzt. Bei ca. 160°C beginnt die Umsetzung der Metalle mit dem Hexanol, erkennbar an der Entwicklung von Wasserstoff und einem Temperaturanstieg auf ca. 230 °C. Man tropft nun mittels eines Tropftrichters weitere 574 g Hexanol über einen Zeitraum von 60 min. zu. Das Reaktionsgemisch wird bei 90°C filtriert. Das Filtrat wird in drei aliquoten Teilen in einer Vorlage bestehend aus 872 g deionisiertem Wasser und darin gelösten 27,8 g Ammoniumcarbonat hydrolysiert. Es entsteht sofort ein weißer Niederschlag. Der überstehende Alkohol wird abdekantiert, geringe Mengen des in der Wasserphase gelösten Alkohols können durch eine Wasserdampfdestillation abgestrippt werden. Der so erhaltene Slurry wird sprühgetrocknet. Die Ausbeute beträgt entsprechend 98 % der Theorie. Die ICP-Analyse ergibt ein Verhältnis Aluminiumoxid:Magnesiumoxid wie 31,8 %:68,2 % (berechnet 30 %:70 %). Die Auswertung der Thermogravimetrie ergibt: Rückstand 56,3 % entsprechend Mg₆Al₂O₉ (theor. 56,9 %), - 4 H₂O (Kristallwasser) - 1 H₂O 15,2 % (theor. 14,9 %), - 6 H₂O - H₂CO₃ 28,7 % (theor. 28,2 %).

### Beispiel 5 Verbindung 12 aus Tabelle 1

In einen 1000 ml Dreihalskolben werden 8 g Aluminium-Nadeln und 21,1 g Magnesium-Granulate vorgelegt. Dazu gibt man 118 g Hexanol. Die Mischung wird erhitzt. Bei ca. 160°C beginnt die Umsetzung der Metalle mit dem Hexanol, erkennbar an der Entwicklung von Wasserstoff und einem Temperaturanstieg auf ca. 230 °C. Man tropft nun mittels eines Tropftrichters weitere 574 g Hexanol über einen Zeitraum von 60 min. zu. Das Reaktionsgemisch wird bei 90°C filtriert. Das Filtrat wird in drei aliquoten Teilen in einer Vorlage bestehend aus 542 g deionisiertem Wasser und darin gelösten 358 g Ammoniummolybdat (NH₄)₆Mo₇O₂₄ hydrolysiert. Es entsteht sofort ein weißer Niederschlag. Der überstehende Alkohol wird abdekantiert, geringe Mengen des in der Wasserphase gelösten Alkohols können durch eine Wasserdampfdestillation abgestrippt werden. Der so erhaltene Slurry wird sprühgetrocknet. Die Ausbeute beträgt entsprechend 98 % der Theorie. Die ICP-Analyse ergibt ein Verhältnis Aluminiumoxid:Magnesiumoxid wie 30,9 %:69,1 % (berechnet 30 %:70 %), der Gehalt an Mo₇O₂₄ beträgt 42,0 % (theor. 41,5 %). Die Auswertung der Thermogravimetrie ergibt: Rückstand 82,1 % % entsprechend Mg₁₈Al₆O₂₇ + Mo₇O₂₄ (theor. 79,0 %), - 12 H₂O (Kristallwasser) - 24 H₂O 21,0 % (theor. 23,7 %).

### Beispiel 6 Verbindung 17 aus Tabelle 1

In einen 1000 ml Dreihalskolben werden 11,3 g Aluminium-Nadeln und 15,1 g Magnesium-Granulate vorgelegt. Dazu gibt man 176 g Hexanol. Die Mischung wird erhitzt. Bei ca. 160°C beginnt die Umsetzung der Metalle mit dem Hexanol, erkennbar an der Entwicklung von Wasserstoff und einem Temperaturanstieg auf ca. 230 °C. Man tropft nun mittels eines Tropftrichters weitere 456 g Hexanol über einen Zeitraum von 60 min. zu. Das Reaktionsgemisch wird bei 90°C filtriert. Das Filtrat wird in drei aliquoten Teilen in einer Vorlage bestehend aus 782 g deionisiertem Wasser und darin gelösten 39,8 g Ammoniumcarbonat hydrolysiert. Es entsteht sofort ein weißer Niederschlag. Der überstehende Alkohol wird abdekantiert, geringe Mengen des in der Wasserphase gelösten Alkohols können durch eine Wasserdampfdestillation abgestrippt werden. Der so erhaltene Slurry wird sprühgetrocknet. Die Ausbeute beträgt entsprechend 98 % der Theorie. Die ICP-Analyse ergibt ein Verhältnis Aluminiumoxid:Magnesiumoxid wie 49,0 %:51,0 % (berechnet 46 %:54 %). Die Auswertung der Thermogravimetrie ergibt: Rückstand 53,0 % entsprechend Mg₃Al₂O₆ (theor. 52,0 %), - 4 H₂O (Kristallwasser) - H₂O 19,9 % (theor. 21,0 %), - 3 H₂O - H₂CO₃ 25,1 % (theor. 27,1 %) .

### Beispiel 7 Verbindung 18 aus Tabelle 1

In einen 1000 ml Dreihalskolben werden 11,3 g Aluminium-Nadeln und 15,1 g Magnesium-Granulate vorgelegt. Dazu gibt man 176 g Hexanol. Die Mischung wird erhitzt. Bei ca. 160°C beginnt die Umsetzung der Metalle mit dem Hexanol, erkennbar an der Entwicklung von Wasserstoff und einem Temperaturanstieg auf ca. 230 °C. Man tropft nun mittels eines Tropftrichters weitere 456 g Hexanol über einen Zeitraum von 60 min. zu. Das Reaktionsgemisch wird bei 90 °C filtriert. Das Filtrat wird in drei aliquoten Teilen in einer Vorlage bestehend aus 729 g deionisiertem Wasser und darin gelösten 92,7 g Ammoniumcitrat (NH₄)₂C₆H₆O₇ hydrolysiert. Es entsteht sofort ein weißer Niederschlag. Der überstehende Alkohol wird abdekantiert, geringe Mengen des in der Wasserphase gelösten Alkohols können durch eine Wasserdampfdestillation abgestrippt werden. Der so erhaltene Slurry wird sprühgetrocknet. Die Ausbeute beträgt entsprechend 98 % der Theorie. Die ICP-Analyse ergibt ein Verhältnis Aluminiumoxid:Magnesiumoxid wie 49,7 %:50,3 % (berechnet 46 %:54 %). Die Auswertung der Thermogravimetrie ergibt: Rückstand 42,9 % entsprechend Mg₃Al₂O₆ (theor. 39,9 %), - 4 H₂O (Kristallwasser) 13,4 % (theor. 12,9 %), - 4 H₂O - C₆H₈O₇ 42,9 % (theor. 38,7 %).

### Beispiel 8 wie Verbindung 6 aus Tabelle 1 aber getrennte Herstellung der Metallalkoholate

In einen 500 ml Dreihalskolben werden 7,9 g Aluminium-Nadeln vorgelegt. Dazu gibt man 50 g Hexanol. Die Mischung wird erhitzt. Bei ca. 160 °C beginnt die Umsetzung des Metalls mit dem Hexanol, erkennbar an der Entwicklung von Wasserstoff und einem Temperaturanstieg auf ca. 230 °C. Man tropft nun mittels eines Tropftrichters weitere 220 g Hexanol über einen Zeitraum von 60 min. zu. Das Reaktionsgemisch wird bei 90 °C filtriert. In einen 500 ml Dreihalskolben werden 21,1 g Magnesium-Granulat vorgelegt. Dazu gibt man 120 g Hexanol. Die Mischung wird erhitzt. Bei ca. 150 °C beginnt die Umsetzung des Metalls mit dem Hexanol, erkennbar an der Entwicklung von Wasserstoff und einem Temperaturanstieg auf ca. 200 °C. Man tropft nun mittels eines Tropftrichters weitere 307 g Hexanol über einen Zeitraum von 60 min. zu. Das Reaktionsgemisch wird bei 90 °C filtriert. Man vereinigt beide Alkoholate. Nun wird in drei aliquoten Teilen in einer Vorlage bestehend aus 943 g deionisiertem Wasser (enthält 0,2 Gew.% Ammoniak) hydrolysiert. Es entsteht sofort ein weißer Niederschlag. Der überstehende Alkohol wird abdekantiert, geringe Mengen des in der Wasserphase gelösten Alkohols können durch eine Wasserdampfdestillation abgestrippt werden. Der so erhaltene Slurry wird sprühgetrocknet. Die Ausbeute beträgt entsprechend 96 % der Theorie. Die ICP-Analyse ergibt ein Verhältnis Aluminiumoxid:Magnesiumoxid wie 32,1%:67,9 % (berechnet 30 %:70 %). Die Auswertung der Thermogravimetrie ergibt: Rückstand 60,1 % entsprechend Mg₆Al₂O₉ (theor. 59,5 %), - 4 H₂O (Kristallwasser) 13,3 % (theor. 12,5 %), - 9 H₂O 26,9 % (theor. 28,0 %).

### Beispiel 9 wie Verbindung 6 aus Tabelle 1 aber Verwendung gemischter Alkohole

In einen 1000 ml Dreihalskolben werden 7,9 g Aluminium-Nadeln und 21,1 g Magnesium-Granulate vorgelegt. Dazu gibt man 120 g eines Gemisches aus Butanol, Hexanol und Octanol wie 1:7:2. Die Mischung wird erhitzt. Bei ca. 150 °C beginnt die Umsetzung der Metalle mit dem Alkoholgemisch, erkennbar an der Entwicklung von Wasserstoff und einem Temperaturanstieg auf ca. 210 °C. Man tropft nun mittels eines Tropftrichters weitere 552 g des Alkoholgemisches über einen Zeitraum von 60 min. zu. Das Reaktionsgemisch wird bei 90 °C filtriert. Das Filtrat wird in drei aliquoten Teilen in einer Vorlage bestehend aus 900 g deionisiertem Wasser (enthält 0,2 Gew.% Ammoniak) hydrolysiert. Es entsteht sofort ein weißer Niederschlag. Der überstehende Alkohol wird abdekantiert, zur Entfernung des Butanols und geringer Mengen des in der Wasserphase gelösten Hexanols und Octanols werden durch eine Wasserdampfdestillation abgestrippt. Der so erhaltene Slurry wird sprühgetrocknet. Die Ausbeute beträgt entsprechend 97 % der Theorie. Die ICP-Analyse ergibt ein Verhältnis Aluminiumoxid:Magnesiumoxid wie 32,2 %:67,8 % (berechnet 30%:70 %). Die Auswertung der Thermogravimetrie ergibt: Rückstand 60,4 % entsprechend Mg₆Al₂O₉ (theor. 59,5 %), - 4 H₂O (Kristallwasser) 13,0 % (theor. 12,5 %), - 9 H₂O 27,1 % (theor. 28,0 %).

### Beispiel 10 Verbindung 19 aus Tabelle 1 Umsetzung eines Metallalkoholates und einem Metall plus Alkohol

In einen 1000 ml Dreihalskolben werden 7,9 g Aluminium-Nadeln vorgelegt. Dazu gibt man 50 g Hexanol. Die Mischung wird erhitzt. Bei ca. 160 °C beginnt die Umsetzung des Metalls mit dem Hexanol, erkennbar an der Entwicklung von Wasserstoff und einem Temperaturanstieg auf ca. 230 °C.

Man tropft nun mittels eines Tropftrichters weitere 220 g Hexanol über einen Zeitraum von 60 min. zu. Hierzu fügt man 115,3 g Zinkdiethanolat. Man läßt die Mischung auf 90°C abkühlen und filtriert. Das Filtrat wird in drei aliquoten Teilen in einer Vorlage bestehend aus 510 g deionisiertem Wasser (enthält 0,2 Gew.% Ammoniak) hydrolysiert. Es entsteht sofort ein weißer Niederschlag. Der überstehende Alkohol wird abdekantiert, geringe Mengen des in der Wasserphase gelösten Alkohols können durch eine Wasserdampfdestillation abgestrippt werden. Der so erhaltene Slurry wird sprühgetrocknet. Die Ausbeute beträgt entsprechend 96 % der Theorie. Die ICP-Analyse ergibt ein Verhältnis Aluminiumoxid:Zinkoxid wie 16,8 %:83,2 % (berechnet 17 %:83 %). Die Auswertung der Thermogravimetrie ergibt: Rückstand 72,2 % entsprechend Zn₆Al₂O₉ (theor. 71,6 %), - 4 H₂O (Kristallwasser) 9,1 % (theor. 8,7 %), - 9 H₂O 19,5 % (theor. 19,7 %).

## Patentansprüche

1. Verfahren zur Herstellung von Hydrotalciten von hoher Reinheit, die anionische, schichtförmig aufgebaute gemischte Metallhydroxide gemäß der allgemeinen Formel
M²⁺₂ₓM³⁺₂(OH)₄ₓ₊₄Aⁿ⁻_{2/n}•zH₂O
sind, worin M²⁺, M³⁺ zwei bzw. dreiwertige Metalle sind, x 0,5 bis 10 in 0,5 Schritten ist, A für ein interstitielles Anion steht, n die Ladung des interstitiellen Anions ist und z eine ganze Zahl von 1 bis 6 ist, **dadurch gekennzeichnet**, daß man
(A) Metallalkoholat-Mischungen einsetzt, die aus zumindest einem oder mehreren zweiwertigen Metallen und zumindest einem oder mehreren dreiwertigen Metallen und aus mono-, di- oder trivalenten C1-bis C40-Alkoholaten bestehen, wobei die zweiwertigen und dreiwertigen Metallalkoholate im wesentlichen in einem molaren Verhältnis eingesetzt werden, das der Stöchiometrie einer Zielverbindung gemäß obiger Summenformel entspricht und
(B) das erhaltene Alkoholatgemisch mit Wasser hydrolisiert, wobei das Wasser zur Hydrolyse überstöchiometrisch in Bezug auf die reaktiven Valenzen der eingesetzten Metalle eingesetzt wird.

2. Verfahren zur Herstellung von Metalloxiden,
**dadurch gekennzeichnet**, daß man die nach dem Verfahren des Anspruchs 1 hergestellten Hydrotalcite kalziniert.

3. Verfahren gemäß Anspruch 1, **dadurch gekennzeichnet**, daß man die Metallalkoholate zuvor durch Umsetzung von Metallen, die die Oxidationstufe +II oder +III ausbilden können, mit den Alkoholen gemäß Anspruch 1 herstellt, wobei die Herstellung der Metallalkoholate
(A) durch gemeinsames Vorlegen der Metalle und Zugabe des Alkohols oder
(B) getrennte Herstellung der Metallalkoholate ggf. mit unterschiedlichen Alkoholat-Resten oder
(C) nacheinander durch Vorlegen des einen Metalls, Zugabe des Alkohols, Zugabe des nächsten Metalls und ggf. erneuter Alkoholzugabe
erfolgt.

4. Verfahren gemäß einem der vorhergehenden Ansprüche, **dadurch gekennzeichnet**, daß als zweiwertige Metalle Mg, Zn, Cu, Ni, Co, Mn, Ca und/oder Fe und als dreiwertige Metalle Al, Fe, Co, Mn, La, Ce und/oder Cr eingesetzt werden.

5. Verfahren gemäß einem der vorhergehenden Ansprüche, **dadurch gekennzeichnet**, daß man vor oder während der Hydrolyse als dritte oder weitere Metallkomponente wasserlösliche zweiwertige und/oder dreiwertige Metalle als Metallsalze zusetzt, wobei die Metallsalze gegenüber den Metallalkoholaten im molaren Unterschuß verwendet werden und das molare Verhältnis der zweiwertigen und dreiwertigen Metalle gemäß Anspruch 1 beibehalten wird.

6. Verfahren gemäß einem der vorhergehenden Ansprüche, **dadurch gekennzeichnet**, daß man für die Hydrolyse Wasser einsetzt, das organische oder anorganische wasserlösliche Anionen enthält.

7. Verfahren gemäß einem der vorhergehenden Ansprüche, **dadurch gekennzeichnet**, daß man für die Hydrolyse Wasser einsetzt, das folgende wasserlösliche Anionen enthält
a. Hydroxid-Anionen und/oder
b. organische Anionen, insbesondere Alkoholate, Alkylethersulfate, Arylethersulfate und/oder Glykolethersulfate und/oder
c. anorganische Anionen, insbesondere Carbonat, Hydrogencarbonat, Chlorid, Nitrat, Sulfat und/oder Polyoxometallanionen,
wobei das Gegenion bevorzugt Ammonium ist und von den Anionen Hydroxid-Ionen bevorzugt sind.

8. Verfahren gemäß einem der vorhergehenden Ansprüche, **dadurch gekennzeichnet**, daß man das Alkoholat/ Alkoholatgemisch vor der Hydrolyse filtriert.

## Claims

1. A process for producing high-purity hydrotalcites which are stratiform, anionic mixed metal hydroxides of the general formula
M²⁺ ₂ₓ M³⁺ ₂ (OH)₄ₓ₊₄ A_{2/n}ⁿ⁻ · z H₂O
wherein
M²⁺ and M³⁺ are divalent and trivalent metals respectively,
X ranges from 0.5 to 10 in intervals of 0.5,
A is an interstitial anion,
n is the charge of said interstitial anion, and
z is an integer of 1 to 6,
characterized in that
(A) metal alcoholate mixtures comprising at least one or more divalent metals, at least one or more trivalent metals, and mono-, di-, or trihydric C₁ - to C₄₀ - alcoholates are used, said di- and trihydric metal alcoholates being substantially used in a molar ratio corresponding to the stoichiometry of any desired compound according the empirical formula referred to hereinabove, and
(B) the resultant alcoholate mixture is hydrolyzed with water, the water for hydrolysis being used in stoichiometric excess, referring to the reactive valences of the metals used.

2. A process for producing metal oxides characterized in that the hydrotalcites produced according to the process of claim 1 are calcinated.

3. A process according to claim 1 characterized in that the metal alcoholates are first produced by reacting metals being capable for providing oxidation numbers +II or +III with the alcohols according to claim 1, the production of said metal alcoholates being accomplished by
(A) placing the metals jointly into the reaction vessel and then adding the alcohol, or
(B) producing the metal alcoholates separately, the alcoholates optionally having different alcoholate residues, or
(C) consecutively by placing one metal into the reaction vessel, adding the alcohol, followed by addition of the second metal, and, optionally, of further amounts of alcohol.

4. A process according to any one of the preceding claims characterized in that as divalent metals
Mg, Zn, Cu, Ni, Co, Mn, Ca and/or Fe
and as trivalent metals
Al, Fe, Co, Mn, La, Ce and/or Cr
are used.

5. A process according to any one of the preceding claims characterized in that prior to or during the hydrolysis, water-soluble, di- and/or trivalent metals as metal salts are added as third or additional metal component, the metal salts being used in stoichiometric quantities smaller than the metal alcoholates, and the molar ratio of the divalent : trivalent metals being as defined in claim 1 hereinabove.

6. A process according to any one of the preceding claims characterized in that water containing water-soluble, organic or inorganic anions is used for the hydrolysis.

7. A process according to any one of the preceding claims characterized in that for hydrolysis water is used which contains any of the following water-soluble anions:
a. hydroxide anions and/or
b. organic anions, particularly alcoholates, alkyl ether sulfates, aryl ether sulfates and/or glycol ether sulfates and/or
c. inorganic anions, particularly carbonate, hydrogen carbonate, chloride, nitrate, sulfate and/or polyoxometal anions,
wherein the counter ion is preferably ammonium and the anions are preferably hydroxide ions.

8. A process according to any one of the preceding claims characterized in that prior to hydrolysis the alcoholate / alcoholate mixture is filtered.

## Revendications

1. Procédé de préparation d'hydrotalcites de pureté élevée, qui sont des hydroxydes métalliques anioniques mixtes à structure stratifiée selon la formule générale
M²⁺ ₂ₓ M³⁺ ₂(OH)₄ₓ₊₄Aⁿ⁻ _{2/n}.zH₂O
dans laquelle M²⁺, M³⁺ sont des métaux divalents ou selon le cas trivalents, x est 0,5 à 10 par pas de 0,5, A représente un anion interstitiel, n est la charge de l'anion interstitiel et z est un nombre entier de 1 à 6, caractérisé en ce que
(A) l'on utilise des mélanges d'alcoolates métalliques qui sont constitués d'au moins un ou de plusieurs métaux divalents et d'au moins un ou de plusieurs métaux trivalents et d'alcoolates mono, di ou trivalents en C₁ à C₄₀, et les alcoolates métalliques divalents et trivalents étant utilisés dans un rapport molaire qui correspond à la stoechiométrie d'un composé visé selon la formule brute ci-dessus, et
(B) le mélange d'alcoolates obtenu est hydrolysé avec de l'eau, l'eau pour l'hydrolyse étant utilisée en quantité au-delà de la stoechiométrie par rapport aux valences réactives des métaux utilisés.

2. Procédé de préparation d'oxydes métalliques, caractérisé en ce que l'on calcine les hydrotalcites obtenus par le procédé selon la revendication 1.

3. Procédé selon la revendication 1, caractérisé en ce que l'on prépare d'abord les alcoolates métalliques par conversion de métaux, qui peuvent former les étages d'oxydation +II ou +III, avec les alcools selon la revendication 1, la préparation des alcoolates métalliques s'effectuant par
(A) introduction commune préalable des métaux et addition de l'alcool, ou
(B) préparation séparée des alcoolates métalliques, ou selon le cas avec différents radicaux alcoolates, ou
(C) successivement, par introduction préalable d'un des métaux, addition de l'alcool, addition du métal suivant et le cas échéant nouvelle addition d'alcool.

4. Procédé selon l'une des revendications précédentes, caractérisé en ce que l'on utilise comme métaux divalents Mg, Zn, Cu, Ni, Co, Mn, Ca et/ou Fe, et comme métaux trivalents Al, Fe, Co, Mn, La, Ce et/ou Cr.

5. Procédé selon l'une des revendications précédentes, caractérisé en ce qu'avant ou pendant l'hydrolyse, on ajoute comme troisième ou comme autres composants métalliques, des métaux divalents et/ou trivalents solubles dans l'eau, sous forme de sels métalliques, les sels métalliques étant utilisés en déficit molaire par rapport aux alcoolates métalliques et le rapport molaire entre les métaux divalents et trivalents selon la revendication 1 étant maintenu.

6. Procédé selon l'une des revendications précédentes, caractérisé en ce que pour l'hydrolyse, on utilise de l'eau qui contient des anions organiques ou minéraux solubles dans l'eau.

7. Procédé selon l'une des revendications précédentes, caractérisé en ce que pour l'hydrolyse, on utilise de l'eau qui contient les anions solubles dans l'eau suivants:
a. anions hydroxyde et/ou
b. anions organiques, en particulier alcoolates, sulfates d'alkyléther, sulfates d'aryléther et/ou sulfates de glycoléther et/ou
c. anions minéraux, en particulier carbonate, hydrogénocarbonate, chlorure, nitrate, sulfate et/ou anions polyoxométalliques,
l'ion complémentaire étant de préférence l'ammonium, et parmi les anions, les ions hydroxyde étant préférés.

8. Procédé selon l'une des revendications précédentes, caractérisé en ce qu'avant l'hydrolyse, on filtre l'alcoolate/mélange d'alcoolates.
